# FASCICULE DE BREVET EUROPEEN

(11) **EP 0 441 689 B1**
(45) Date de publication et mention de la délivrance du brevet: **14.12.1994**
(21) Numéro de dépôt: 91400254.8
(22) Date de dépôt: 04.02.1991
(51) Int. Cl.: A61K 7/13, A61K 7/06, A61K 7/48, C12P 17/10, C09B 69/10

(54) **Procédé de préparation d'un pigment mélanique par voie enzymatique et son utilisation en cosmétique**
Verfahren zur enzymatischen Herstellung von Melaninpigment und seine kosmetische Verwendung
Process for enzymatic preparation of melanin pigment and its use in cosmetics

(30) Priorité: 05.02.1990 LU 87672
(43) Date de publication de la demande: 14.08.1991
(73) Titulaire: L'OREAL, 75008 Paris (FR)
(72) Inventeur: Junino, Alex, F-93180 Livry-Gargan (FR); Andrean, Hervé, F-75014 Paris (FR); Tuloup, Rémi, F-35190 Miniac-sous-Bécherel (FR); Higgins, Irwing John, Graze Hill Bedford MK 44 2TF (GB)
(74) Mandataire: Casalonga, Axel

(56) Documents cités:
- EP-A- 0 313 380
- GB-A- 2 207 153
- TETRAHEDRON, vol. 44, no. 23, 1988, pages 7265-7270, Pergamon Press Plc, GB; A.NAPOLITANO et al.: "A profile of the oxidation chemistry of 5-hydroxyindole under biomimetic conditions"
- CHEMICAL ABSTRACTS, vol. 78, no. 16, 23 avril 1973, page 6, résumé no. 98091p,Columbus, Ohio, US; E. FATTORUSSO et al.: "Polymerization process of 5,6-dihydroxyindole", & REND. ACCAD. SCI. FIS. MAT.,NAPLES 1971, 38, 173-82

## Description

L'invention est relative à un nouveau procédé de préparation de pigments mélaniques par voie enzymatique, ainsi que leur utilisation, notamment en cosmétique.

L'utilisation de pigments colorés présente un très grand intérêt dans le domaine cosmétique.

Il s'agit essentiellement de pigments minéraux ou de pigments issus de colorants directs de synthèse, ou de carbone pur dans le cas de pigments noirs.

Ces différents produits peuvent présenter des problèmes de mise en ouvre et certains d'entre eux ne sont pas totalement inoffensifs.

On sait qu'il est possible de produire des colorants jaune-bruns par oxydation du 5-hydroxyindole avec la peroxydase de raifort en présence d'eau oxygénée. Ce colorant est constitué de dimères et de trimères du 5-hydroxyindole (A. NAPOLITANO et Coll. TETRAHEDRON, Vol. 44 n° 23, pages 7265-7270 - 1988).

De même, chemi Abst. Vol 78 n° 16 (1973) n° 98091 p décrit l'oxydation d'un composé où la position 5 est occupé par un hydroxyle.

On sait également qu'il est possible de déméthyler la 9-méthoxy-ellipticine par la peroxydase de raifort en présence d'eau oxygénée, pour former principalement la 9-oxo-ellipticine (Gérard MEUNIER et Coll., J. Am. Chem. Soc., 1985, 107, 2558-2560). Ce procédé permet d'obtenir la quinone-imine avec des rendements d'au moins 90%.

Le document EP 313 380 décrit, comme dérivé indolique précurseur de la mélanine, le 5-hydroxyindole, et l'obtention de pigments par oxydation de latyrosinax.

Le document GB 2 207 153 décrit la préparation de poudres contenant des pigments mélaniques résultant de l'oxydation de composés indoliques en présence d'agents oxydants tels que les iodures, periodades, permanganates, persulfates et dichromates.

La demanderesse a découvert de façon surprenante, qu'il était possible de préparer des pigments mélaniques par voie enzymatique, par polymérisation oxydative de dérivés du 5,6-dihydroxyindole dont les fonctions hydroxyle sont bloquées. Ces dérivés du 5,6-dihydroxyindole ont l'avantage d'être stables et d'un coût relativement bas.

Le pigment obtenu par ce procédé est incorporé plus aisément dans les compositions cosmétiques du fait de sa facilité de dispersion et de sa faible granulométrie.

L'objet de l'invention est donc constitué par un procédé de préparation d'un pigment mélanique et le pigment ainsi obtenu.

On appelle "pigment mélanique", le pigment résultant de la polymérisation oxydative de précurseurs indoliques; ce pigment est identique ou similaire à la mélanine.

D'autres objets de l'invention apparaîtront à la lecture de la description et des exemples qui suivent.

Le procédé de préparation du pigment mélanique est essentiellement caractérisé par le fait que l'on polymérise un composé répondant à la formule : 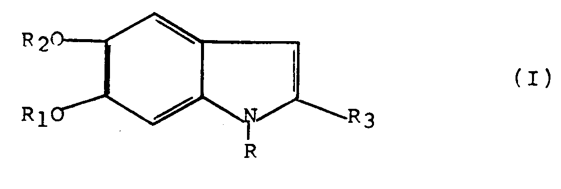
dans laquelle :
R représente un atome d'hydrogène, un radical alkyle, alcoxy, hydroxyalkyle, aminoalkyle, SiR₄R₅R₆ où R₄,R₅,R₆ désignent un groupement alkyle, dans lesquels le reste alkyle contient de 1 à 8 atomes de carbone ou un radical aryle non substitué ou substitué par OH, NH₂, alkyle, alcoxy ou NO₂;
R₁ et R₂, identiques ou différents, représentent des groupements alkyle ou forment ensemble un groupement méthylène ou éthylène, éventuellement substitué par un ou plusieurs groupements alkyle, R₃ représente un atome d'hydrogène ou le radical COOH, en présence d'un milieu oxydant et d'une enzyme à activité (per)oxydante ou d'un composé ayant une activité similaire.

Les radicaux alkyle ou alcoxy désignent de préférence des radicaux inférieurs ayant 1 à 6 atomes de carbone; aryle désigne de préférence phényle.

Les composés répondant à la formule (I) ci-dessus indiquée, sont choisis de préférence parmi le 5,6-diméthoxyindole, le 5,6-méthylènedioxyindole et le 1-méthyl 5,6-diméthoxyindole.

Le milieu oxydant peut être constitué par des peroxydes tels que le peroxyde d'hydrogène ou des persels tels que le monopersulfate de potassium (oxone), par l'oxygène de l'air, suivant la nature des enzymes à activité oxydante mis en oeuvre.

Ces enzymes, à activité peroxydante, sont choisies parmi la peroxydase de raifort, la chloroperoxydase, la peroxydase du lait, la cytochrome C-peroxydase, la microperoxydase.

Les composés ayant une activité similaire à celle des enzymes à activité peroxydante, sont choisis par l'hémoglobine brute ou purifiée, la myoglobine, la méthémoglobine et la métmyoglobine.

Les enzymes à activité oxydante sont choisies parmi les alcool-oxydases, tels que la méthanol-oxydase, les polyphénoloxydases, ou encore les mono-oxygénases de déalkylation comme les enzymes produites par le micro-organisme PSEUDOMONAS TESTOSTERONI. (D.W. RIBBONS, FEBS Letters, 8 (1970), p.101)).

La peroxydase de raifort, l'hémoglobine et la méthémoglobine sont particulièrement préférées.

Dans le cas de l'hémoglobine, on peut ajouter au milieu réactionnel du ferricyanure de potassium pour la transformer en méthémoglobine.

Lorsqu'on utilise l'hémoglobine, la myoglobine, la méthémoglobine ou la métmyoglobine, on peut ajouter au milieu réactionnel un sel minéral comme le sulfate d'ammonium ou un produit organique tel que le formamide ou la guanidine sous forme de chlorhydrate.

La concentration en peroxyde d'hydrogène utilisé avec les enzymes à activité peroxydante est faible et généralement inférieure à 5% en poids par rapport au poids total du milieu réactionnel et de préférence inférieure à 2%. La concentration minimale étant de 0,007%.

La concentration en persel est comprise entre 2 et 600 millimoles/litre.

Dans le cas de la méthanol-oxydase, on l'utilise en présence de méthanol ou d'éthanol et l'oxygène de l'air comme oxydant.

Le rapport en poids entre le dérivé indolique de formule (I) et l'enzyme à activité oxydante ou le composé à activité similaire peut varier dans de larges proportions allant de 1 à 10⁶.

La préparation du pigment mélanique s'effectue généralement à un pH compatible avec les enzymes mis en oeuvre et est compris généralement entre 2 et 11, et de préférence entre 4 et 8. Ce pH est obtenu à l'aide d'un tampon approprié à l'enzyme considéré, tel que par exemple par un tampon acétate, un tampon phosphate ou un tampon citrate.

La température de réaction est compatible avec les enzymes utilisés et elle est comprise généralement entre 10°C et 50°C, et de préférence entre 20 et 45°C.

Le milieu réactionnel est généralement constitué par de l'eau ou un mélange eau-solvant organique. Dans certains cas, ce milieu peut être constitué uniquement par un solvant organique lorsqu'on utilise d'autres hydroperoxydes que le peroxyde d'hydrogène.

Les solvants utilisables sont choisis notamment parmi les hydrocarbures aromatiques tels que le toluène, ou des esters tels que l'acétate d'éthyle, ou encore le diméthylformamide, la N-méthylpyrrolidone, le diméthylsulfoxyde, le dioxane, l'acétone.

La formation du pigment mélanique doit être effectuée dans des conditions telles à avoir une bonne dispersion de l'enzyme dans le milieu réactionnel. On peut ainsi procéder par immobilisation de l'enzyme sur la surface d'un support approprié ou encore utiliser une enzyme modifiée chimiquement de façon à faciliter sa solubilisation dans le milieu réactionnel.

Une forme de réalisation préférée consiste à immobiliser la peroxydase de raifort selon la technique de séchage à l'air de l'enzyme sur une surface de billes de verre, comme décrit dans le Journal of American Chemical Society, 107, 5448, KAZANTJIAN, R.Z. and KLIBANOV, 1985.

Dans un mode de réalisation préféré, on ajoute à une solution de précurseur indolique de formule (I), dans un milieu solvant aqueux, une enzyme à activité peroxydante et après 1 à 30 minutes, on additionne une solution de peroxyde d'hydrogène. Après 1 à 24 heures, on sépare un précipité brun à noir par filtration, centrifugation ou lyophilisation. Ce précipité est éventuellement lavé à l'eau ou avec un solvant organique tels que ceux cités précédemment, puis séché.

Les pigments selon l'invention peuvent être caractérisés par RPE (résonance paramagnétique électronique) à l'aide d'un spectromètre ER 200 D BRUCKER à 9,52 GHZ avec une fréquence de modulation de champ de 100 KHz et une puissance de micro-onde de 1,9 mw. On enregistre l'absorption de cette onde par le pigment en fonction du champ et on constate une absorption maximale vers 3480 Gauss. Ce maximum d'absorption se retrouve dans les eumélanines naturelles.

Le pigment mélanique ainsi préparé, peut être utilisé dans des applications diverses où l'on souhaite pouvoir disposer d'un pigment brun à noir, tel que notamment en cosmétique dans les produits de maquillage, les compositions solaires, les produits de coloration capillaire.

Ils peuvent être introduits en vue de ces applications dans un milieu cosmétiquement acceptable à base d'eau ou de mélanges eau-solvant(s) organique(s) ou d'un ou plusieurs solvants, et éventuellement d'autres additifs habituellement utilisés en cosmétique, tels que des agents tensio-actifs, épaississants, conservateurs, ...

Selon une variante de l'invention, le pigment mélanique peut être déposé sur une charge minérale constituée de particules inertes ayant une granulométrie inférieure à 20 µm et de préférence 10 µm. Le pigment peut également être déposé et/ou absorbé sur une charge organique constituée de particules de polymères ayant une granulométrie inférieure à 100 µm ou sur un matériau polymérique réticulé formant des microsphères, comme décrit dans la demande EP 313.380.

Selon une autre variante, le pigment peut être déposé et/ou absorbé sur des particules, organiques ou minérales, à structure lamellaire, ayant une dimension inférieure à 50 µm.

On obtient ainsi une poudre colorée dans des nuances allant du brun au noir, qui peut être utilisée en cosmétique.

Selon cette variante, cette poudre colorée peut être préparée en dispersant les particules minérales ou organiques dans la solution de précurseur indolique de formule (I) contenant l'enzyme. Les conditions d'oxydation du précurseur de formule (I) sont identiques à celles décrites ci-dessus et notamment dans le cas où l'on utilise une enzyme ou un composé à activité similaire à activité peroxydante, la solution contient en outre du peroxyde d'hydrogène ou un persel.

Après 1 minute à 24 heures de réaction, on sépare par filtration la poudre contenant le pigment, on la lave à l'eau ou avec un solvant organique et on la sèche.

Cette poudre peut également être préparée par absorption du pigment mélanique préparé conformément à l'invention, sur et dans les particules minérales ou organiques définies ci-dessus.

On procède notamment en dispersant le pigment mélanique formé au préalable dans un milieu non solvant des particules et contenant lesdites particules, et qu'après absorption du pigment, on les sèche.

On utilise plus particulièrement comme charge minérale des particules de carbonate de calcium ou de silice ayant une granulométrie généralement supérieure à 0,01 µm.

Comme charge organique, on utilise de préférence des particules de polymères dérivés de la kératine éventuellement modifiée, de polymères dérivés de la chitine éventuellement désacétylée, de fibroïne de soie, de polymères synthétiques choisis parmi le polyméthacrylate de méthyle réticulé, la poly-β-alanine réticulée, des microsphères creuses de copolymère chlorure de vinylidène et acrylonitrile, ou des microsphères poreuses de polyamide-12, polyamide-6 ou de copolyamide 6/12, de poudres de silicones constituées par des gommes, des résines, des élastomères d'organo-polysiloxanes.

Ces particules ont une granulométrie de préférence supérieure à 0,01 µm.

Comme matériau polymérique réticulé, on peut citer les microsphères de styrène/divinylbenzène, méthacrylate de méthyle/diméthacrylate d'éthylèneglycol, dont la granulométrie est de préférence comprise entre 5 et 100 µm.

Comme particules, organiques ou- minérales, à structure lamellaire, on utilise de préférence la L-lauroyllysine, les microparticules de céramique éventuellement recouvertes de poudre de zirconium, le dioxyde de titane lamellaire, le talc lamellaire, le nitrure de bore, le mica, l'oxychlorure de bismuth. Ces particules ont une granulométrie de préférence supérieure à 0,5 µm et inférieure à 50 µm, le rapport entre la plus grande dimension et l'épaisseur étant compris entre 2 et 100.

Les exemples qui suivent sont destinés à illustrer l'invention sans pour autant présenter un caractère limitatif.

### EXEMPLE DE PREPAPATION 1

### Préparation d'un pigment mélanique à partir du 5,6-diméthoxyindole.

On ajoute 0,378 g de peroxydase de raifort (Sigma) et 1 g de 5,6-diméthoxyindole dans 8 ml de Diméthylformamide (DMF) à 4 litres d'un tampon acétate de sodium (20 nMoles-pH 5).

15 minutes après l'addition de l'enzyme, on ajoute 8 ml d'eau oxygénée à 110 volumes.

Le mélange réactionnel est abandonné 6 heures sous agitation, puis une nuit au repos à température ambiante. Après centrifugation, puis lavage à l'eau, le pigment noir est séché sous vide.

L'analyse RPE montre une absorption maximale vers 3480 Gauss.

### EXEMPLE DE PREPAPATION 2

### Préparation d'un pigment mélanique à partir du 5,6-méthylènedioxyindole.

On ajoute 0,6 g de peroxydase de raifort (Sigma) et 1,6 g de 5,6-méthylènedioxyindole dans 13 ml de diméthylformamide à 6,4 l d'un tampon acétate de sodium (20 nMoles-pH 5).

15 minutes après l'addition de l'enzyme, on ajoute 12,8 ml d'eau oxygénée à 110 volumes.

Le milieu réactionnel est abandonné 36 heures à température ambiante. Après centrifugation, puis lavage à l'eau, le pigment noir obtenu est séché sous vide.

L'analyse RPE montre une absorption maximale vers 3480 Gauss.

### EXEMPLE DE PREPARATION 3

### Préparation d'un pigment mélanique à partir du 5,6-diméthoxyindole.

Après dissolution à température ambiante, sous agitation, de 1 g d'hémoglobine commerciale vendue par la société SIGMA, dans 50 cm³ de solution tampon 0,1 M de citrate de sodium-acide citrique, ajustée au pH=5,5, on ajoute 30 g de sulfate d'ammonium. Après 1/2 heure d'agitation, la solution est centrifugée 20 minutes à 10.000 tours/minute. L'hémoglobine déposée est reprise par 500 cm³ de solution tampon 0,1 M d'acétate de sodium-acide acétique, ajustée au pH=5 et amenée à une température de 37°C. On ajoute dans la solution 60 mg de ferricyanure de potassium, puis 1/2 heure après 1 g de 5,6-diméthoxyindole dissous dans 4 cm³ de diméthylformamide. Sous vive agitation, sont alors versés 4 cm³ d'eau oxygénée à 110 volumes.

Le mélange réactionnel est abandonné 4 heures à 37°C sous agitation puis est centrifugé. La couleur du pigment est verdâtre. Le pigment noir est obtenu par lavages à la diméthylformamide, à l'acétone puis à l'eau et est séché sous vide.

L'analyse RPE montre une absorption maximale vers 3480 gauss.

### EXEMPLE DE PREPARATION 4

### Préparation d'un pigment mélanique à partir du 5,6-diméthoxyindole.

1 g d'hémoglobine commerciale vendue par la Société SIGMA est dissous dans 100 cm³ d'eau distillée, sous agitation à température ambiante. Sont versés successivement après dissolution, 1 g de 5,6-diméthoxyindole dans 4 cm³ de diméthylformamide, puis 3,4 g d'oxone (monopersulfate de potassium, triple sel) en poudre. Le mélange réactionnel est abondonné 5 minutes à température ambiante sous agitation puis est centrifugé. La couleur du pigment est verdâtre. Le pigment noir est obtenu par lavages à la diméthylformamide, à l'acétone, puis à l'eau et est séché sous vide.

L'analyse RPE montre une absorption maximale vers 3480 gauss.

### EXEMPLE DE PREPARATION 5

### Préparation d'un pigment mélanique à partir du 5,6-diméthoxyindole.

1 g d'hémoglobine commerciale vendue par la Société SIGMA est dissous dans 500 cm³ de tampon acétate de sodium-acide acétique, ajusté au pH=5 et contenant 0,1 g de formamide (ou 0,1 g de chlorhydrate de guanidinium). Après 1 heure d'agitation à 37°C, on ajoute successivement 1 g de 5,6-diméthoxyindole dans 4 cm³ de DMF puis 4 cm³ d'eau oxygénée à 110 volumes. Le mélange réactionnel est abandonné 4 heures à 37°C sous agitation puis est centrifugé. Le pigment noir obtenu est lavé à la DMF, à l'acétone puis à l'eau et est séché sous vide.

L'analyse RPE montre un absorption maximale vers 3480 gauss.

### EXEMPLE DE PREPARATION 6

### Préparation d'un pigment mélanique à partir du 5,6-diméthoxyindole.

1 g d'hémoglobine commerciale vendue par la Société SIGMA est dissous dans 500 cm³ de tampon acétate de sodium-acide acétique, ajusté au pH=5. On ajoute successivement 1 g de 5,6-diméthoxyindole dans 4 cm³ de DMF puis 4 cm³ d'eau oxygénée à 110 volumes. Le mélange réactionnel est abandonné 4 heures à 37°C sous agitation puis est centrifugé. Le pigment brun obtenu est lavé à la DMF, à l'acétone puis à l'eau et est séché sous vide.

L'analyse RPE montre une absorption maximale vers 3480 gauss.

### EXEMPLE DE PREPARATION 7

### Préparation d'un pigment mélanique à partir du 1-méthyl 5,6-diméthoxyindole.

Après dissolution à température ambiante, sous agitation, de 1 g d'hémoglobine commerciale vendue par la Société SIGMA, dans 50 cm³ de solution tampon 0,1 M de citrate de sodium-acide citrique, ajustée au pH=5,5, on ajoute 30 g de sulfate d'ammonium. Après 1/2 heure d'agitation, la solution est centrifugée 20 minutes à 10.000 tours /minute. L'hémoglobine déposée est reprise par 500 cm³ de solution tampon 0,1 M d'acétate de sodium-acide acétique, ajustée au pH=5 et amenée à une température de 37°C. On ajoute à la solution 60 mg de ferricyanure de potassium. Le milieu est abandonné 1/2 heure sous vive agitation puis est additionné successivement de 1 g de 1-méthyl 5,6-diméthoxyindole dissous dans 4 cm³ de DMF puis de 4 cm³ d'eau oxygénée à 110 volumes.

Le mélange réactionnel est abandonné 4 heures à 37°C sous agitation puis est centrifugé. Le pigment brun obtenu est lavé à la DMF, à l'acétone puis à l'eau et est séché sous vide.

L'analyse RPE montre une absorption maximale vers 3480 gauss.

### EXEMPLES D'APPLICATION

### EXEMPLE A : MASCARA CREME

| | |
|---|---|
| - Pigment noir obtenu selon l'exemple 1 | 15,0 g |
| - Stéarate de triéthanolamine | 15,0 g |
| - Cire de Candellila | 8,0 g |
| - Cire de Carnauba | 10,0 g |
| - Hydroxyéthylcellulose | 0,9 g |
| - Hydrolysat de kératine (exprimé en matière sèche) | 0,75 g |
| - Conservateurs qs | |
| - Eau | qsp 100,0 g |

On peut utiliser le pigment obtenu à l'exemple 3 à la place de celui obtenu à l'exemple 1.

### EXEMPLE B : GEL CAPILLAIRE

| | |
|---|---|
| - Pigment noir obtenu selon l'exemple 2 | 0,5 g |
| - Copolymère de vinylpyrrolidone/acétate de vinyle, vendu sous la dénomination PVP/VA S 630 par la Société GAF | 1,5 g |
| - Alcool éthylique | 15,0 g |
| - Carbopol 940 (GOODRICH CHEMICAL) | 0,7 g |
| - Triéthanolamine qs pH = 7,5 | |
| - Conservateurs qs | |
| - Eau | qsp 100,0 g |

On peut utiliser le pigment obtenu à l'exemple 5 à la place de celui obtenu à l'exemple 2.

## Revendications

1. Procédé de préparation d'un pigment mélanique, caractérisé, par le fait que l'on polymérise un composé répondant à la formule : 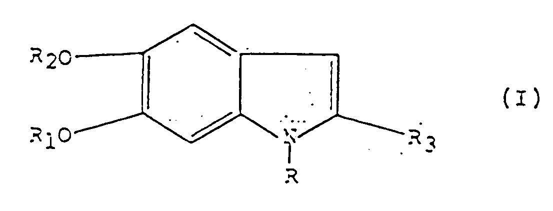 dans laquelle :
R représente un atome d'hydrogène, un radical alkyle, alcoxy, hydroxyalkyle, aminoalkyle, SiR₄R₅R₆ où R₄,R₅,R₆ désignent alkyle, dans lesquels le reste alkyle comporte de 1 à 8 atomes de carbone ou un radical aryle non substitué ou substitué par OH, NH₂, alkyle, alcoxy ou NO₂;
R₁ et R₂, identiques ou différents, représentent des groupements alkyle en C₁-C₆ ou forment ensemble un groupement méthylène ou éthylène, éventuellement substitué par un ou plusieurs groupements alkyle inférieur, R₃ représente hydrogène ou COOH, en présence d'un milieu oxydant et
au moins
- une enzyme à activité (per)oxydante choisie parmi la peroxydase de Raifort, la chloroperoxydase, la peroxydase du lait, la cytochrome C-peroxydase ou la microperoxydase,
- un composé choisi parmi l'hémoglobine, la myoglobine, la méthémoglobine et la métmyoglobine,
- une enzyme à activité oxydante choisie parmi les alcool-oxydases, les polyphénol-oxydases et les mono-oxygénases de déalkylation.

2. Procédé selon la revendication 1, caractérisé par le fait que les composés de formule (I) sont choisis parmi le 5,6-diméthoxyindole, le 5,6-méthylènedioxyindole et le 1-méthyl 5,6-diméthoxyindole.

3. Procédé selon l'une quelconque des revendications 1 ou 2, caractérisé par le fait que les enzymes à activité peroxydante sont choisies parmi la peroxydase de raifort, la chloroperoxydase, la peroxydase du lait, la cytochrome C-peroxydase ou la microperoxydase.

4. Procédé selon les revendications 3, caractérisé par le fait que le milieu oxydant est constitué par du peroxyde d'hydrogène ou un persel.

5. Procédé selon l'une quelconque des revendications 1 à 4, caractérisé par le fait que l'on procède à la polymérisation oxydative du composé de formule (I), en présence de peroxyde d'hydrogène et de peroxydase de raifort.

6. Procédé selon l'une quelconque des revendications 1, 2, 4, caractérisé par le fait que l'on procède à la polymérisation oxydative du composé de formule (I), en présence de peroxyde d'hydrogène ou d'oxone et d'hémoglobine.

7. Procédé selon la revendication 1 ou 2, caractérisé par le fait que les enzymes à activité oxydante sont choisies parmi les alcool-oxydases, les polyphénol-oxydases et les mono-oxygénases de déalkylation et que le milieu oxydant est constitué par l'oxygène de l'air.

8. Procédé selon la revendication 7, caractérisé par le fait que les alcool-oxydases sont choisies parmi les méthanol-oxydases.

9. Procédé selon l'une quelconque des revendications 1 à 8, caractérisé par le fait que le rapport en poids entre le dérivé indolique de formule (I) et l'enzyme à activité (per)oxydante ou composé ayant une activité similaire, est compris entre 1 et 10⁶.

10. Procédé selon l'une quelconque des revendications 1 à 6 et 9, caractérisé par le fait que la concentration en peroxyde d'hydrogène dans le milieu réactionnel est inférieure à 5%.

11. Procédé selon l'une quelconque des revendications 1 à 10, caractérisé par le fait que le milieu réactionnel est constitué par de l'eau, un mélange eau-solvant organique et que le pH est maintenu à une valeur comprise entre 2 et 11, à l'aide d'un tampon approprié à l'enzyme considérée.

12. Procédé selon l'une quelconque des revendications 1 à 11, caractérisé par le fait que la température de réaction est comprise entre 10 et 50°C, et de préférence entre 20 et 45°C.

13. Procédé selon l'une quelconque des revendications 1 à 12, caractérisé par le fait que l'on procède par immobilisation de l'enzyme sur la surface d'un support.

14. Procédé de préparation d'une poudre colorée constituée de particules minérales de granulométrie inférieure à 20 µm ou organiques de granulométrie inférieure à 100 µm, à structure lamellaire ou non ou de particules de polymères organiques ayant une granulométrie inférieure à 100 µm ou de particules d'un matériau polymérique réticulé ayant une granulométrie comprise entre 5 et 100 µm, recouverte de pigment mélanique ou comportant un pigment mélanique, caractérisé par le fait que l'on disperse les particules minérales ou les particules organiques ou le matériau réticulé ou les particules à structure lamellaire dans la solution des précurseurs indoliques de formule (I) qui est oxydé selon le procédé tel que défini dans l'une quelconque des revendications 1 à 12 ou que l'on prépare le pigment mélanique selon l'une des revendications 1 à 12 et l'on absorbe sur et dans les particules minérales et/ou organiques.

15. Procédé de préparation d'une poudre colorée constituée de particules de polymères organiques ayant une granulométrie inférieure à 100 µm ou d'un matériau polymérique réticulé ayant une granulométrie comprise entre 5 et 100 µm comportant un pigment mélanique, caractérisé par le fait qu'on disperse les particules organiques ou le matériau réticulé dans la solution de précurseur indolique de formule (I) qui est oxydé selon le procédé tel que défini dans l'une quelconque des revendications 1 à 12.

16. Procédé de préparation d'une poudre colorée constituée de particules minérales ayant une granulométrie inférieure à 20 µm ou de particules organiques ayant une granulométrie inférieure à 100 µm caractérisé par le fait que le pigment mélanique préparé selon l'une quelconque des revendications 1 à 12, est absorbé sur et dans les particules minérales et/ou organiques.

17. Procédé de préparation d'une poudre colorée constituée de particules minérales ou organiques, à structure lamellaire, recouverte de pigment mélanique, caractérisé par le fait qu'on disperse les particules à structure lamellaire dans la solution de précurseur indolique de formule (I) qui est oxydé selon le procédé tel que défini dans l'une quelconque des revendications 1 à 12.

## Claims

1. Process for the preparation of a melanic pigment, characterized in that a compound is polymerized which corresponds to the formula: 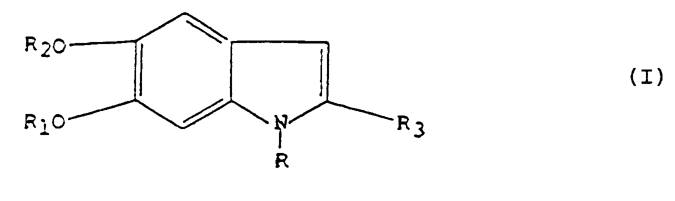 in which:
R denotes a hydrogen atom or an alkyl, alkoxy, hydroxyalkyl or SiR₄R₅R₆ radical, where R₄, R₅ and R₆ denote alkyl or aminoalkyl, in which the alkyl residue contains from 1 to 8 carbon atoms, or an aryl radical which is unsubstituted or substituted by OH, NH₂, alkyl, alkoxy or NO₂;
R₁ and R₂, which are identical or different, denote C₁-C₆ alkyl groups or together form a methylene or ethylene group which is optionally substituted by one or more lower alkyl groups, and R₃ denotes hydrogen or COOH, in the presence of an oxidizing medium and
at least
- an enzyme with (per)oxidizing activity chosen from horseradish peroxidase, chloroperoxidase, milk peroxidase, cytochrome C peroxidase or microperoxidase,
- a compound chosen from haemoglobin, myoglobin, methaemoglobin and metmyoglobin,
- an enzyme with oxidizing activity chosen from alcohol-oxidases, polyphenol-oxidases and dealkylating mono-oxygenases.

2. Process according to Claim 1, characterized in that the compounds of formula (I) are chosen from 5,6-dimethoxyindole, 5,6-methylenedioxyindole and 1-methyl-5,6-dimethoxyindole.

3. Process according to either of Claims 1 and 2, characterized in that the enzymes with (per)oxidizing activity are chosen from horseradish peroxidase, chloro-peroxidase, milk peroxidase, cytochrome C peroxidase or microperoxidase.

4. Process according to Claims [sic] 3, characterized in that the oxidizing medium consists of hydrogen peroxide or a persalt.

5. Process according to any one of Claims 1 to 4, characterized in that the oxidative polymerization of the compound of formula (I) is performed in the presence of hydrogen peroxide and of horseradish peroxidase.

6. Process according to any one of Claims 1, 2, 4, characterized in that the oxidative polymerization of the compound of formula (I) is performed in the presence of hydrogen peroxide or of oxone and of haemoglobin.

7. Process according to Claim 1 or 2, characterized in that the enzymes with oxidizing activity are chosen from alcohol-oxidases, polyphenol-oxidases and dealkylating monooxygenases and in that the oxidizing medium consists of atmospheric oxygen.

8. Process according to Claim 7, characterized in that the alcohol-oxidases are chosen from methanol-oxidases.

9. Process according to any one of Claims 1 to 8, characterized in that the weight relationship between the indole-related derivative of formula (I) and the enzyme with (per)oxidizing activity or compound which has a similar activity is between 1 and 10⁶.

10. Process according to any one of Claims 1 to 6 and 9, characterized in that the concentration of hydrogen peroxide in the reaction mixture is lower than 5%.

11. Process according to any one of Claims 1 to 10, characterized in that the reaction medium consists of water or a mixture of water and organic solvent and in that the pH is maintained at a value of between 2 and 11 with the aid of a buffer which is appropriate to the enzyme in question.

12. Process according to any one of Claims 1 to 11, characterized in that the reaction temperature is between 10 and 50°C, and preferably between 20 and 45°C.

13. Process according to any one of Claims 1 to 12, characterized in that the procedure involves immobilizing the enzyme on the surface of a support.

14. Process for the preparation of a coloured powder consisting of inorganic particles smaller than 20 µm in particle size or organic ones smaller than 100 µm in particle size, with lamellar or other structure, or of particles of organic polymers with a particle size smaller than 100 µm or of particles of a crosslinked polymeric material with a particle size of between 5 and 100 µm, covered with melanic pigment or comprising a melanic pigment, characterized in that the inorganic particles or the organic particles or the crosslinked material or the particles with lamellar structure are dispersed in the solution of the indolic precursors of formula (I), which is oxidized according to the process as defined in any one of Claims 1 to 12 or in that the melanic pigment is prepared according to one of Claims 1 to 12 and is absorbed onto and into the inorganic and/or organic particles.

15. Process for the preparation of a coloured powder consisting of particles of organic polymers which have a particle size smaller than 100 µm or of a crosslinked polymeric material which has a particle size of between 5 and 100 µm, comprising a melanic pigment, characterized in that the organic particles or the crosslinked material are dispersed in the solution of indole-related precursor of formula (I) which is oxidized according to the process as defined in any one of Claims 1 to 12.

16. Process for the preparation of a coloured powder consisting of inorganic particles which have a particle size smaller than 20 µm or of organic particles which have a particle size smaller than 100 µm, characterized in that the melanic pigment prepared according to any one of Claims 1 to 12 is absorbed onto and into the inorganic and/or organic particles.

17. Process for the preparation of a coloured powder consisting of inorganic or organic particles, with lamellar structure, covered with melanic pigment, characterized in that the particles with lamellar structure are dispersed in the solution of indole-related precursor of formula (I) which is oxidized according to the process as defined in any one of Claims 1 to 12.

## Patentansprüche

1. Verfahren zur Herstellung eines Melaninpigments
dadurch **gekennzeichnet**, daß
man eine Verbindung der Formel: 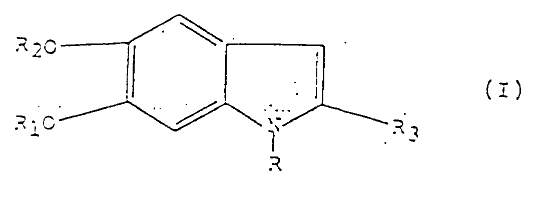 worin gilt:
R stellt ein Wasserstoffatom, einen Alkyl-, Alkoxy-, Hydroxyakyl-, Aminoalkyl-, -SiR₄R₅R₆-Rest, worin R₄, R₅ und R₆ eine Alkylgruppe bedeuten, in denen der Alkylrest 1 bis 8 Kohlenstofatome enthält, oder einen Arylrest dar, der nicht substituiert oder mit einer -OH-, -NH₂-, Alkyl-, Alkoxy- oder -NO₂-Gruppe substituiert ist;
R₁ und R₂ stellen, gleich oder verschieden, C₁₋₆-Alkylgruppen dar oder bilden zusammen eine Methylen- oder Ethylengruppe, die gegebenenfalls mit einer oder mehreren Niedrigalkylgruppen substituiert sind;
R₃ stellt ein Wasserstoffatom oder den -COOH-Rest dar,
in Gegenwart eines oxidierenden Mediums und mindestens
- eines Enzyms mit (per)oxidierender Wirksamkeit, ausgewählt aus Peroxidase von Raifort, Chlorperoxidase, Peroxidase der Milch, Cytochrom C-Peroxidase oder Mikroperoxidase,
- einer Verbindung, ausgewählt aus Hämoglobin, Myoglobin, Methämoglybin und Metmyoglobin,
- eines Enzyms mit oxidierender Wirksamkeit, ausgewählt aus Alkohol-Oxidasen, Polyphenol-Oxidasen und Dealkylierungs-Monooxigenasen,
polymerisiert.

2. Verfahren gemäß Anspruch 1,
dadurch **gekennzeichnet**, daß
die Verbindungen der Formel (I) aus 5,6-Dimethoxyindol, 5,6-Methylendioxyindol und 1-Methyl-5,6-dimethoxyindol ausgewählt sind.

3. Verfahren gemäß einem jeden der Ansprüche 1 oder 2,
dadurch **gekennzeichnet**, daß
die Enzyme mit peroxidierender Wirksamkeit aus Peroxydase von Raifort, Chlorperoxidase, Peroxidase der Milch, Cytochrom-C-Peroxidase oder Mikroperoxidase ausgewählt sind.

4. Verfahren gemäß Anspruch 3,
dadurch **gekennzeichnet**, daß
das oxidierende Medium durch Wasserstoffperoxid oder ein Persalz dargestellt ist.

5. Verfahren gemäß einem jeden der Ansprüche 1 bis 4,
dadurch **gekennzeichnet**, daß
man die Verbindung der Formel (I) in Gegenwart von Wasserstoffperoxid und von Peroxidase von Raifort oxidativ polymerisiert.

6. Verfahren gemäß jedem der Ansprüche 1, 2 und 4,
dadurch **gekennzeichnet**, daß
man die Verbindung der Formel (I) in Gegenwart von Wasserstoffperoxid oder Oxon sowie von Hämoglobin oxidativ polymerisiert.

7. Verfahren gemäß Anspruch 1 oder 2,
dadurch **gekennzeichnet**, daß
die Enzyme mit oxidierender Wirksamkeit aus Alkohol-Oxidasen, Polyphenol-Oxidasen und Dealkylierungs-Monoxigenasen ausgewählt und das oxidierende Medium durch Luftsauerstoff dargestellt sind.

8. Verfahren gemäß Anspruch 7,
dadurch **gekennzeichnet**, daß
die Alkohol-Oxidasen aus Methanol-Oxidasen ausgewählt sind.

9. Verfahren gemäß jedem der Ansprüche 1 bis 8,
dadurch **gekennzeichnet**, daß
das Gewichtsverhältnis des Indolderivats der Formel (I) zum Enzym mit (per)oxidierender Wirksamkeit oder der Verbindung mit einer entsprechenden Wirksamkeit 1 bis 10⁶ beträgt.

10. Verfahren gemäß jedem der Ansprüche 1 bis 6 und 9,
dadurch **gekennzeichnet**, daß
die Konzentration an Wasserstoffperoxid im Reaktionsmedium unterhalb 5% liegt.

11. Verfahren gemäß einem jeden der Ansprüche 1 bis 10,
dadurch **gekennzeichnet**, daß
das Reaktionsmedium durch Wasser, eine Mischung aus Wasserorganisches Lösungsmittel dargestellt ist und der pH mit einem für das in Betracht gezogene Enzym geeigneten Puffer bei einem Wert von 2 bis 11 gehalten wird.

12. Verfahren gemäß jedem der Ansprüche 1 bis 11,
dadurch **gekennzeichnet**, daß
die Reaktionstemperatur 10 bis 50°C, vorzugsweise 20 bis 45°C, beträgt.

13. Verfahren gemäß einem jeden der Ansprüche 1 bis 12,
dadurch **gekennzeichnet**, daß
man das Enzym auf der Oberfläche einer Trägerunterlage immobilisiert.

14. Verfahren zur Herstellung eines gefärbten Pulvers aus mineralischen Partikeln mit einer Korngröße unterhalb 20 µm oder aus organischen Partikeln mit einer Korngröße unterhalb 100 µm, mit lamellarer Struktur oder nicht, oder aus Partikeln von organischen Polymeren mit einer Korngröße unterhalb 100 µm oder aus Partikeln eines vernetzten Polymermaterials mit einer Korngröße von 5 bis 100 µm, welche mit Melaninpigment bedeckt sind oder ein Melaninpigment enthalten,
dadurch **gekennzeichnet**, daß
man die mineralischen Partikel oder die organischen Partikel oder das vernetzte Material oder die Partikel mit lamellarer Struktur in einer Lösung indolischer Vorstufen der Formel (I) dispergiert, welche gemäß dem in einem jeden der Ansprüche 1 bis 12 definierten Verfahren oxidiert werden, oder daß man das Melaninpigment gemäß einem der Ansprüche 1 bis 12 herstellt und auf oder in den mineralischen und/oder organischen Partikeln absorbiert.

15. Herstellungsverfahren eines gefärbten Pulvers aus Partikeln von organischen Polymeren mit einer Korngröße unterhalb 100 µm oder aus Partikeln eines vernetzten Polymermaterials mit einer Korngröße von 5 bis 100 µm, welche ein Melaninpigment enthalten,
dadurch **gekennzeichnet**, daß
man die organischen Partikel oder das vernetzte Material in einer Lösung indolischer Vorstufen der Formel (I) dispergiert, welche gemäß dem in einem jeden der Ansprüche 1 bis 12 definierten Verfahren oxidiert werden.

16. Verfahren zur Herstellung eines gefärbten Pulvers aus mineralischen Partikeln mit einer Korngröße unterhalb 20 µm oder aus organischen Partikeln mit einer Korngröße unterhalb 100 µm,
dadurch **gekennzeichnet**, daß
das gemäß einem jeden der Ansprüche 1 bis 12 hergestellte Melaninpigment auf und in den mineralischen und/oder organischen Partikeln absorbiert wird.

17. Verfahren zur Herstellung eines gefärbten Pulvers aus mineralischen oder organischen Partiklen, mit lamellarer Struktur, welche mit Melaninpigment bedeckt sind,
dadurch **gekennzeichnet**, daß
man die Partikel mit lamellarer Struktur in einer Lösung indolischer Vorstufen der Formel (I) dispergiert, welche gemäß dem in einem jeden der Ansprüche 1 bis 12 definierten Verfahren oxidiert werden.
